# EUROPEAN PATENT APPLICATION

(11) **EP 1 746 164 A1**
(43) Date of publication of application: **24.01.2007**
(21) Application number: 05734395.6
(22) Date of filing: 20.04.2005
(51) Int. Cl.: C12P 19/18, C07H 3/06, C12P 19/14, A23C 9/12, A23G 1/00, A23L 1/22, A23L 1/29, C12R 1/865, C12R 1/07

(54) **SUGAR WITH HIGH CONTENT OF LACTOSUCROSE, PROCESS FOR PRODUCING THE SAME AND USE THEREOF**

(30) Priority: 22.04.2004 JP 2004126305; 02.08.2004 JP 2004226109; 10.08.2004 JP 2004232937
(71) Applicant: KABUSHIKI KAISHA HAYASHIBARA SEIBUTSU KAGAKU KENKYUJO, Okayama-shi, Okayama 700-0907 (JP)
(72) Inventor: OKABE, Hiroyuki, KABUSHIKI KAISHA HAYASHIBARA, Okayama-shi, Okayama 7000907 (JP); AGA, H., K.K. HAYASHIBARA SEIBUTSU KAGAKUKENKYOJO, Okayama-shi, Okayama 7000907 (JP); KUBOTA, M., K.K. HAYASHIBARA SEIBUTSU KAGAKU KEN., Okayama-shi, Okayama 7000907 (JP); MIYAKE, T., K.K. HAYASHIBARA SEIBUTSU KAGAKU KEN., Okayama-shi, Okayama 7000907 (JP)
(74) Representative: Daniels, Jeffrey Nicholas
(86) International application number: PCT/JP2005/007528
(87) International publication number: WO 2005/103276

(57) **Abstract**

An object of the present invention is to provide a lactosucrose high content saccharide which comprises 70% or more of lactosucrose, on a saccharide composition basis, with a lower content of 1-kestose and fructosyllactosucrose as by-products. Another object of the present invention is to provide processes for producing a lactosucrose high content saccharide and high purity lactosucrose containing 90% or more of lactosucrose, on a saccharide composition basis, which are feasible for industrial production. Further object of the present invention is to provide a solid lactosucrose with low hygroscopicity and a solid composition comprising the same.

The present invention solves above objects by providing a lactosucrose high content saccharide comprising 70% or more of lactosucrose and less than 3% of the total amount of 1-kestose and fructosyl lactosucrose, on a saccharide composition basis; a process for producing the lactosucrose high content saccharide, comprising the steps of allowing β-fructofuranosidase, derived from a microorganism belonging to the genus *Bacillus,* and sucrose-unassimilable yeast to contact with an aqueous solution containing sucrose and lactose to obtain a reaction mixture comprising 70% or more of lactosucrose and less than 3% of the total amount of 1-kestose and fructosyl lactosucrose and collecting the resulting lactosucrose high content saccharide; a process for producing a high purity lactosucrose, comprising the steps of subjecting the lactosucrose high content saccharide to a chromatography using a resin and collecting fractions containing 90% or more of lactosucrose, on a saccharide composition basis; a process for producing crystalline lactosucrose; and various solid compositions prepared by incorporating the crystalline lactosucrose.

## Description

### TECHNICAL FIELD

The present invention relates to a lactosucrose high content saccharide, its preparation and uses, more particularly, to a lactosucrose high content saccharide comprising 70% or more of lactosucrose and less than 3% of the total amount of 1-kestose and fructosyl lactosucrose, on a saccharide composition basis, and its preparation. The present invention further relates to a process for producing a high purity lactosucrose, comprising the steps of subjecting a lactosucrose high content saccharide to chromatography using a resin and collecting fractions containing 90% or more of lactosucrose, on a saccharide composition basis; and to a process for producing crystalline lactosucrose.

### BACKGROUND ART

Recently, there has been an increase of knowledge on functions of various saccharides represented by oligosaccharides. Among functional oligosaccharides, lactosucrose [β-D-galactosyl-(1,4)-α-D-glucosyl-(1,2)-β-D-fructoside] was revealed to have satisfactory functions such as low digestivity, bifidobacteria-growth-promoting activity, low cariogenicity and moisture-retaining ability and usefulness in various fields such as foods, cosmetics and pharmaceuticals. Lactosucrose is industrially produced by a saccharide-transferring action catalyzed by β-fructofuranosidase, by allowing β-fructofuranosidase derived from a microorganism of the genus *Arthrobacter* to act on an aqueous solution containing sucrose and lactose (see Japanese Patent Kokai No. 27, 285/91). However, in the case of using the glycosyl-transferring reaction of β-fructofuranosidase alone, lactosucrose content of the resulting reaction mixture is relatively low, about 30%, on a saccharide composition basis. In addition, the reaction mixture comprises a relatively large amount of unreacted sucrose and lactose, hydrolysates such as glucose and fructose, and by-products such as 1-kestose and fructosyl lactosucrose. Therefore, such saccharide composition can not exhibit inherent functions of lactosucrose sufficiently. A saccharide comprising lactosucrose ("*NYU-KA* OLIGO®") is produced by the methods of upgrading the purity of lactosucrose such as a method of crystallizing unreacted lactose partially from the reaction mixture after the reaction and removing the resulting crystalline lactose by filtration; or adding invertase-deficient yeast to the reaction mixture during the reaction to assimilate monosaccharides such as glucose (see Koki Fujita, "Handbook of Bio-Separation Process", pp.196-201, published by Kyoritsu Shuppan Co., Ltd., 1996). Particularly, the method of using sucrose-unassimilable yeast in combination with glucosyl-transferring reaction by β-fructofuranosidase has the merit of elevating the formation of lactosucrose because the glycosyl-transferring reaction proceeds toward the formation of lactosucrose by eliminating monosaccharides in addition to the mere elimination of monosaccharides such as glucose from the reaction mixture. Therefore, by the method, lactosucrose content is reached about 65%, on a saccharide composition basis (see Japanese Patent Kokai No.293,494/92). Further, a purified lactosucrose with a purity of about 99% can be obtained by the steps of subjecting the reaction mixture with a high content of lactosucrose as a material to chromatography using an ion-exchange resin to elevate the purity of lactosucrose to about 80% and subjecting the resulting lactosucrose high content saccharide to a chromatography using octadecyl silica gel (ODS) (see Koki Fujita, "Handbook of Bio-Separation Process", pp. 196-201, published by Kyoritsu Shuppan Co., Ltd., 1996).

However, β-fructofuranosidase derived from a microorganism of the genus *Arthrobacter* catalyzes 1-kestose-forming reaction by transferring a fructosyl residue to sucrose and fructosyl lactosucrose-forming reaction by transferring a fructosyl residue to the formed lactosucrose as side reactions except for lactosucrose-forming reaction by transferring a fructosyl residue of sucrose to lactose. The formation of 1-kestose and fructosyl lactosucrose as by-products is not preferable from the viewpoint of the production of lactosucrose and the lowering of the lactosucrose content in the reaction mixture. Since the above by-products are not assimilated by invertase-deficient yeast, they accumulate in the reaction mixture obtained by the method of using β-fructofuranosidase and invertase-deficient yeast simultaneously, and their total content reaches 5 to 10%. Further, in the case of producing a high purity lactosucrose by using the reaction mixture comprising the by-products, 1-kestose and fructosyl lactosucrose, as a material, multiple purification means such as chromatographies using the above ion-exchange resin and octadecyl silica gel (ODS) are required. Further, such production has the demerit of resulting in a low yield of a high purity lactosucrose obtained by such purification. Therefore, it is considered that such reaction mixture is not preferable for the industrial production of a high purity lactosucrose.

While, it has been known that a powdery saccharide, comprising lactosucrose with a lowered hygroscopicity and relatively high stability, can be obtained by elevating the purity of lactosucrose (see Japanese Patent Kokai No. 281,795/92). However, subsequent studies revealed that such powdery saccharide easily absorbs moisture and shows low stability and handleability under the conditions of a high temperature and humidity in summer, for example, at 27° C and a relative humidity of 75%. Under these circumstances, the provision of a more stable solid lactosucrose has been desired.

Crystalline lactosucrose was disclosed in Gad Avigad, Journal Biological Chemistry, 1957, vol. 229, pp.121-129, as crystalline lactosucrose pentahydrate obtained from 90% (v/v) of ethanol solution. However, since the crystal showed strong hygroscopicity and the crystallization was difficult, the production of crystalline lactosucrose on an industrial scale has not been established yet.

### DISCLOSURE OF INVENTION

Under these circumstances, an object of the present invention is to provide a lactosucrose high content saccharide which comprises 70% or more of lactosucrose, on a saccharide composition basis, with a lower content of 1-kestose and fructosyl lactosucrose as by-products. Another object of the present invention is to provide a process for producing a lactosucrose high content saccharide and a high purity lactosucrose containing 90% or more of lactosucrose, on a saccharide composition basis, which are feasible for industrial production. Further object of the present invention is to provide a solid lactosucrose with low hygroscopicity and a solid composition comprising the same.

To solve the above objects, the present inventors studied on a process for producing lactosucrose, using β-fructofuranosidase, from the viewpoints of the origin of β-fructofuranosidase and reaction condition as well as the combination with sucrose-unassimilable yeast. It has been thought that the lactosucrose content in the reaction mixture reaches maximum about 65%, on a saccharide composition basis, even in the case of using β-fructofuranosidase and invertase-deficient yeast. Contrary to expectation, however, it was revealed that the lactosucrose content in the reaction mixture reached 70% or more, on a saccharide composition basis, by allowing β-fructofuranosidase derived from a microorganism of the genus *Bacillus,* disclosed in Japanese Patent Kokai No. 224,665/97 applied for by the same applicant as the present invention, and sucrose-unassimilable yeast to contact with a solution containing sucrose and lactose; and controlling the pH of the reaction mixture to pH 4.0 to 5.5. Further it was also revealed that the total content of by-products, 1-kestose and fructosyl lactosucrose, in the reaction mixture was remarkably low, less than 3%, on a saccharide composition basis. In addition, it was revealed that a high purity lactosucrose can be produced easily and in a high yield by purifying the reaction mixture as material by using chromatography. Also, the present inventors found that crystalline lactosucrose (pentahydrate) can be easily obtained from a supersaturated lactosucrose solution with a low content of by-products, 1-kestose and fructosyl lactosucrose. It was also revealed that the resulting crystalline lactosucrose was a solid product which showed a low hygroscopicity under the conditions of a high temperature and humidity in summer, for example, at 27° C and a relative humidity of 75%, and a satisfactory handleability; and can be advantageously used for producing various solid products comprising lactosucrose.

The present invention solves above objects by providing a lactosucrose high content saccharide comprising 70% or more of lactosucrose and less than 3% of the total amount of 1-kestose and fructosyl lactosucrose, on a saccharide composition basis; a process for producing the lactosucrose high content saccharide, comprising the steps of allowing β-fructofuranosidase, derived from a microorganism belonging to the genus *Bacillus,* and sucrose-unassimilable yeast to contact with an aqueous solution containing sucrose and lactose to obtain a reaction mixture comprising 70% or more of lactosucrose and less than 3% of the total amount of 1-kestose and fructosyl lactosucrose and collecting the resulting lactosucrose high content saccharide; a process for producing a high purity lactosucrose, comprising the steps of subjecting the lactosucrose high content saccharide to chromatography using a resin and collecting fractions containing 90% or more of lactosucrose, on a saccharide composition basis; a process for producing crystalline lactosucrose; and various solid compositions prepared by incorporating the crystalline lactosucrose.

According to the present invention, a lactosucrose high content saccharide comprising 70% or more of lactosucrose and less than 3% of the total of by-products, 1-kestose and fructosyl lactosucrose, on a saccharide composition basis, can be produced from sucrose and lactose by a lactosucrose-forming reaction using β-fructofuranosidase and sucrose-unassimilable yeast, without using purification means such as column chromatography. Further, a high purity lactosucrose comprising 90% or more of lactosucrose, on a saccharide composition basis, cab be easily produced by subjecting the lactosucrose high content saccharide to chromatography using a resin. Also, according to the present invention, it is possible that lactosucrose, whose crystallization has been difficult, is easily crystallized from its supersaturated solution. Since the resulting crystalline lactosucrose shows low hygroscopicity under the condition of a high temperature and humidity in summer and a satisfactory handleability, various solid compositions with low hygroscopicity and satisfactory stability can be provided by incorporating the crystalline lactosucrose into solid materials.

### BEST MODE FOR CARRYING OUT THE INVENTION

The lactosucrose high content saccharide according to the present invention comprises 70% or more of lactosucrose and less than 3% of the total of 1-kestose and fructosyl lactosucrose, on a saccharide composition basis. The lactosucrose high content saccharide is characterized in that the contents of 1-kestose and fructosyl lactosucrose, which are formed by side reaction of β-fructofuranosidase, are remarkably low in comparison with conventional saccharides comprising lactosucrose.

The lactosucrose high content saccharide according to the present invention contains unreacted sucrose and lactose, lactulose formed from lactose by isomerization under alkaline condition, glucose and fructose formed from sucrose by hydrolysis of β-fructofuranosidase, and glycerol formed from glucose and fructose by fermentation of sucrose-unassimilable yeast, except for lactosucrose, 1-kestose and fructosyl lactosucrose. As described later in Examples, the major saccharide in these is lactose because it is not hydrolyzed by β-fructofuranosidase and not assimilated and eliminated by sucrose-unassimilable yeast.

The process for producing a lactosucrose high content saccharide of the present invention (hereinafter, maybe simply abbreviated as "the process of the present invention" or "the process") is characterized in that β-fructofuranosidase derived from a microorganism of the genus *Bacillus* and a sucrose-unassimilable yeast are used in combination. The term "β-fructofuranosidase" as referred to in the present invention means an enzyme catalyzing the hydrolysis of β-fructofuranosidic linkage of sucrose, raffinose and erlose to release fructose and the fructosyl transferring reaction from these saccharides having β-fructofuranosidic linkage as a fructosyl donor to an acceptor selected from the group consisting of other saccharides except for fructosyl donor, sugar alcohols and alcohols. The β-fructofuranosidase usable in the present invention is defined as above and not specifically restricted by its preparation method as far as it is derived from a microorganism of the genus *Bacillus,* and in the case of using it in combination with a sucrose-unassimilable yeast in the present invention described later in detail, it can be used for producing a lactosucrose high content saccharide comprising 70% or more of lactosucrose and less than 3% of the total of 1-kestose and fructosyl lactosucrose, on a saccharide composition basis. For example, natural β-fructofuranosidase derived from *Bacillus* sp. V230 (FERM BP-5054), disclosed in Japanese Patent Kokai No. 224,665/97 applied for by the same applicant as the present invention, and a recombinant β-fructofuranosidase prepared by recombinant DNA technology using a DNA encoding the above β-fructofuranosidase, disclosed in Japanese Patent Kokai No. 66,586/98 applied for by the same applicant as the present invention can be advantageously used in the present invention. In the case of using a recombinant enzyme, β-fructofuranosidase obtained from a host cell except for a microorganism of the genus *Bacillus* can be used rightly. Mutated enzymes obtained by applying protein-engineering technology on a DNA encoding β-fructofuranosidase, disclosed in the same application, can be used in the present invention as far as they do not substantially lose their prescribed transferring activities.

The term "sucrose-unassimilable yeast" as referred to in the present invention means yeast which is capable of assimilating monosaccharides but not capable of assimilating or hydrolyzing oligosaccharides including disaccharides. The sucrose-unassimilable yeast usable in the present invention is not specifically restricted by its genus as far as it is capable of assimilating glucose and fructose and not capable of assimilating and/or hydrolyzing sucrose, lactose and lactosucrose. For example, yeast which is constructed by mutating yeast, isolated from natural sources, using various mutagens to delete invertase or sucrose-assimilating ability can also be used. Yeasts such as *Saccharomyces cerevisiae* ATCC56741 and ATCC56742, known as invertase-deficient yeast, can be advantageously used in the present invention.

Sucrose and lactose used as materials in the process of the present invention are not specifically restricted by their preparation methods and forms. For example, preparations including commercially available products, which are isolated from natural sources, prepared enzymatically, and synthesized chemically, can be used. Further, preparations comprising other concomitants except for the above saccharides and compositions prepared by using the above preparations in combination can be used.

A reaction product comprising 70% or more of lactosucrose and less than 3% of the total of by-products, 1-kestose and fructosyl lactosucrose, on a saccharide composition basis, can be obtained by carrying out the reaction under arbitral conditions according to the enzymatic properties of β-fructofuranosidase and the physiological properties of sucrose-unassimilable yeast, except for allowing β-fructofuranosidase derived from a microorganism of the genus *Bacillus* and a sucrose-unassimilable yeast to act in combination on an aqueous solution containing sucrose and lactose (hereinafter, either or both of sucrose and lactose maybe abbreviated as "substrate(s)" and controlling the reaction pH to 4.0 to 5.5. The lactosucrose content in the reaction product obtained by the lactosucrose-forming reaction only is usually 70% or more but less than 80%, on a saccharide composition basis, and not elevated to 80% or more. In the case of using β-fructofuranosidase derived from a microorganism of the genus *Bacillus,* disclosed in Japanese Patent Kokai No. 224,665/97 applied for by the same applicant as the present invention, and sucrose-unassimilable *Saccharomyces cerevisiae* ATCC56741 to the lactosucrose-forming reaction, conditions are preferably controlled to those of not inactivating the enzyme completely and allowing the yeast to assimilate fructose; i.e. , the reaction temperature is preferably controlled, usually, in a range of about 0 to 40°C, desirably, in a range of about 15 to 35°C; the pH of the reaction mixture is preferably controlled to, usually, 4.0 to 5.5. The substrate concentration of the reaction mixture is not specifically restricted as far as the prescribed reaction can be proceeded and gives a reaction product comprising 70% or more of lactosucrose and less than 3% of the total of by-products, 1-kestose and fructosyl lactosucrose. However, the concentrations of sucrose and lactose are preferably set to, usually, a range of 0.1 to 40% (w/w), desirably, in a range of 1 to 30% (w/w), respectively. The ratio of sucrose and lactose is preferably set to 1:0.65 to 1.3.

The amount of β-fructofuranosidase is preferably in a range of, usually, 0.1 to 50 units, desirably, 0.5 to 10 units per gram-dry solid of sucrose in reaction materials (substrates). One unit of β-fructofuranosidase activity as referred to as in the present invention is defined as the amount of enzyme which increases the reducing power corresponding to 2 µmol of D-glucose per minute when reacted with sucrose as a substrate at pH 6.0 and 40°C according to the method described in Japanese Patent Kokai No. 224,665/97 applied for by the same applicant as the present invention.

The amount of sucrose-unassimilable yeast cell is preferable in a range of, usually, 0.01 to 1 gram, desirably, 0.05 to 0.2 gram per gram-dry solid of sucrose in reaction materials (substrates).

The operating method of using β-fructofuranosidase and a sucrose-unassimilable yeast in combination is not specifically restricted as far as the reaction product, comprising 70% or more of lactosucrose and less than 3% of the total of by-products, 1-kestose and fructosyl lactosucrose, on a saccharide composition basis, can be obtained. For example, β-fructofuranosidase and yeast can be added to an aqueous solution containing sucrose and lactose simultaneously or at different timing, and either or both of β-fructosfuranosidase and yeast can be arbitrarily supplied. Further, the reaction of β-fructofuranosidase and treatment with sucrose-unassimilable yeast can be carried out in respective reaction vessels, and respective reaction mixture can be circulated for the continuous reaction. In such a case, a bioreactor using immobilized β-fructofuranosidase and sucrose-unassimilable yeast can be advantageously used.

The time for the reaction using β-fructofuranosidase and sucrose-unassimilable yeast can be arbitrarily selected according to the operating method and the degree of the progress of the reaction. The reaction time is preferably set to, usually, 2 to 200 hours, desirably, 10 to 80 hours.

Optionally, β-fructofuranosidase and sucrose-unassimilable yeast, used for the reaction can be advantageously reused after isolating and recovering from the reaction mixture by the methods such as centrifugation and filtration using a membrane.

According to the process for producing a lactosucrose high content saccharide of the present invention, a reaction product comprising 70% or more of lactosucrose and less than 3% of the total of by-products, 1-kestose and fructosyl lactosucrose, on a saccharide composition basis, as a lactosucrose high content saccharide. Such lactosucrose high content saccharide can be used intact. Also, the remaining lactose in the lactosucrose high content saccharide can be partially isomerized into lactulose by alkaline treatment to reduce the content of lactose for inhibiting the crystallization of lactose. Further, the lactosucrose high content saccharide can be purified to the desired level by conventional methods selected from a group consisting of decolorization using activated charcoal, deionization using ion-exchange resins, removing of impurities by insolubilization by saturating carbon dioxide gas, removing of lactose by crystallization, diatomite filtration, concentration using a evaporator,and spray-drying. The purified lactosucrose high content saccharide can be used for various uses.

Further, a syrupy high purity lactosucrose can be produced by the steps of purifying the lactosucrose high content saccharide by chromatography using an ion-exchange resin and collecting fractions containing 90% or more of lactosucrose, on a saccharide composition basis. A powdery or granular high purity lactosucrose product can be produced from a syrupy high purity lactosucrose by an arbitral drying process selected from a group consisting of spray-drying, drying *in vacuo,* and freeze-drying. These syrupy, powdery and granule products can be used for various uses.

Crystalline lactosucrose can be advantageously produced by the steps of preparing a supersaturated lactosucrose solution by using the above lactosucrose high content saccharide or high purity lactosucrose, crystallizing lactosucrose, and collecting the resulting crystalline lactosucrose. The condition of the crystallization is not specifically restricted as far as the material is a supersaturated lactosucrose solution and capable of crystallizing lactosucrose, and usually, an aqueous solution with a lactosucrose concentration of 75% (w/w) or higher is preferable. More concretely, a saccharide comprising lactosucrose with a lactosucrose purity of, usually, about 75% or more, desirably, about 80 to 95% is prepared into a solution with a concentration of about 75 to 95% (w/w) ormore. The temperature of the saccharide solution is controlled to that not freezing the solution but lower than the melting point of crystalline lactosucrose and not causing the browning and decomposition of lactosucrose, for example, at about 20 to 60°C, to crystallize lactosucrose. For the crystallization, an organic solvent such as methanol, ethanol and acetone can be arbitrarily added to the solution for controlling the degree of supersaturation and viscosity.

The massecuite containing crystalline lactosucrose is preferably prepared by the steps of placing a supersaturated solution comprising lactosucrose in a relatively high temperature into a crystallizer; adding small amount of, desirably, 0.1 to 20% (w/w) of seed crystal to the solution; and gradually cooling during a gentle stirring to accelerate crystallization.

The method for producing crystalline lactosucrose from the resultingmassecuite is not restricted as far as crystalline lactosucrose can be obtained and selected from conventional methods such as centrifugation, block-pulverizing, fluidized granulation, and spray-drying.

The centrifugal method is usually carried out using a basket-type centrifuge machine for separating crystalline lactosucrose and syrup. Since the resulting crystals can be easily washed with a small amount of cold water, the centrifugal method is preferable for producing a high purity crystalline lactosucrose. Since syrup is not separated from crystals by other three methods, they can not be used for elevating the purities. However, when the three methods are used for the production, product can be obtained in a higher yield. Therefore, the crystalline lactosucrose products produced by such three methods usually contain other saccharides, derived from material or process, such as sucrose and lactose.

In the case of the spray-drying method, a powdery product comprising crystalline lactosucrose with a no or less hygroscopicity can be easily produced by the steps of spraying a massecuite containing about 20 to 50% (w/w) of crystals from a nozzle using a high-pressure pump; drying the obtained crystals in a warm air at a temperature of not melting the powdery crystals, for example, about 40 to 60°C; and ageing them in a warm air.

In the case of the block-pulverization method, a powdery product comprising crystalline lactosucrose with a no or less hygroscopicity can be easily produced by the steps of allowing a massecuite containing about 10 to 60% (w/w) of crystals for about 0.5 to 5 days to crystallize into a block-form solid; pulverizing the resulting block by pulverization or cutting; and drying crystals.

Further, a solid comprising crystalline lactosucrose with a no or less hygroscopicity can be arbitrarily produced by the steps of concentrating a solution containing lactosucrose by heating to give a moisture content of less than 5% (w/w) according to conventional method to make into a supersaturated lactosucrose solution in a melted form; and directly shaping into various shapes such as a powder, granule, stick, plate, and cube form.

The lactosucrose high content saccharide, high purity lactosucrose, and crystalline lactosucrose can be advantageously used similarly as in conventional lactosucrose and a saccharide comprising lactosucrose, for example, in various fields of foods and beverages, including healthy foods and beverages, cosmetics, pharmaceuticals, and feeds as a sweetener, low-digestive sweetener, low cariogenic sweetener, moisture-retaining agent, starch retrogradation-preventing agent, antiflatulent, mineral absorption-promoting agent, and the like. Particularly, crystalline lactosucrose has, in addition to the above functions, a substantially non-hygroscopicity, and satisfactory fluidity and handleability without fear of adhesion and solidification. Therefore, crystalline lactosucrose enables a serious labor cost reduction for the management for packing, transporting and preserving the product.

Since the crystalline lactosucrose of the present invention is a powder with a substantially non- or less hygroscopicity, having a satisfactory thermal tolerance and stability, various solid compositions such as foods and beverages, cosmetics, pharmaceuticals and shaped products can be produced by incorporating the crystalline lactosucrose, desirably 1% (w/w) or more, into, for example, a powdery mixed sweetener, chocolate, chewing gum, instant juice, instant soup, granule, tablet, and the like as a solidification-preventing agent, excipient, filler, base for powderization, and the like. Further, the crystalline lactosucrose of the present invention can be advantageously used as a reagent, material for chemical industry, and the like.

Further, the crystalline lactosucrose of the present invention can be advantageously used for producing various solid compositions by mixing with other crystalline saccharides, for example, maltose, lactose, maltitol, trehalose, palatinose, sucrose, and the like in a rate of 0.01 to 100-folds to the weight of lactosucrose.

A granular product with a satisfactory fluidity and handleability can be produced by granulating a powdery high purity lactosucrose or crystalline lactosucrose, obtained by the above method, by using an arbitral water-soluble polymer such as pullulan, water-soluble pullulan ether, water-soluble pullulan ester, gelatin, Arabic gum, carboxymethyl cellulose, and seaweed polysaccharide, as a binder.

The following examples explain the present invention in detail.

### Example 1

### Production of a lactosucrose high content saccharide

### Example 1-1

### Preparation of β-fructofuranosidase

According to the method described in Japanese Patent Kokai No. 224,665 applied for by the same applicant as the present invention, *Bacillus* sp. V230 (FERM BP-5054) was cultivated in a culture medium containing sucrose as a carbon source in a 40-L culture scale. Successively, according to the method described in the above application, the resulting culture was centrifuged and about 38 L of the supernatant was collected. Then, β-fructofuranosidase activity of the culture supernatant was confirmed by the assay of β-fructofuranosidase.

The above culture supernatant was concentrated by UF-membrane and 360 ml of crude enzyme solution showing the β-fructofuranosidase activity of about 360 units/ml was obtained. According to the method described in the above application, the crude enzyme was subjected to ion-exchange chromatography using "DEAE-TOYOPEARL" gel and hydrophobic chromatography using "BUTY-TOYOPEARL" gel, both commercialized by Tosoh Corporation, Tokyo, Japan, and then subjected to 2nd ion-exchange chromatography using "DEAE-TOYOPEARL" gel to obtain a purified β-fructofuranosidase preparation showing a single band by 7.5% (w/v) polyacrylamide gel electrophoresis. The specific activity of the purified enzyme preparation was about 205 units/mg-protein.

### Example 1-2

### Cultivation of a sucrose-unassimilable yeast and preparation of the wet cell

Two hundred milliliters of a liquid culture medium consisting of 2.0% (w/v) of glucose, 2.0% (w/v) of yeast extract and water was placed in a 500-ml Elrenmeyer flask, sterilized by autoclaving at 121° C for 20 minutes and cooled. Then, *Saccharomyces cerevisiae* ATCC56741 was inoculated in the culture medium and cultivated at 27° C for 24 hours under 230 rpm rotary-agitation to make into a seed culture. The same culture medium was placed into a 30-L fermenter, sterilized by heating, and then cooled to 27° C. To the resulting culture medium, 1% (w/v) of the seed culture was inoculated and cultivated at 27°C for 48 hours under agitation and aeration condition with keeping the pH 4.0 to 7.0. After the cultivation, about 680 g-wet of yeast cells were collected from the culture by centrifuging at 8,000 rpm for 20 minutes.

### Example 1-3

### Reaction for forming lactosucrose

An aqueous solution containing 20% (w/w) each of sucrose and lactose was adjusted to pH 6.0, and then one unit/g-sucrose of the purified β-fructofuranosidase preparation, prepared in Example 1-1, and 6.9% wet weight/dry-solid of the yeast cells, prepared in Example 1-2 were admixed with the aqueous solution and followed by the enzyme reaction at 30°C for 24 and 48 hours with controlling the pH 4.0 to 5.5 using 1 N of aqueous sodium hydroxide solution. After the reaction, saccharide composition of the reaction mixture was analyzed by HPLC. HPLC was carried out under the following condition: column, "TSK-GEL AMIDO-80", commercialized by Tosoh Corporation, Tokyo, Japan; column temperature, 35°C; eluent, acetonitrile : water (71:29); flow rate, 1.0 ml/min; detection, differential refractometer "RI-8012", commercialized by Tosoh Corporation, Tokyo, Japan. The results of saccharide composition analyses were in Table 1. As a reference, β-fructofuranosidase originated from *Arthrobacter* sp. K-1 was purified according to the method described in Koki Fujita et al. , Agric. Biol. Chem. , vol. 54, pp. 913-919 (1990) and the same reaction and analysis were carried out except for using the enzyme of 10 units/g-sucrose and controlling the pH of the reaction mixture to pH 5.5 to 6.5. The results were in Table 1.

**Table 1**

| Origin of enzyme | Reaction Time (h) | Saccharide composition (%) | | | | | | | |
|---|---|---|---|---|---|---|---|---|---|
| | | Gly | DP1 | Suc | Lac | Kes | LS | F-LS | UN |
| V230 (This invention) | 24 | 1.2 | 0.5 | 8.8 | 17.3 | 0.1 | 71.0 | 0.0 | 1.1 |
| | 42 | 1.8 | 0.0 | 0.6 | 22.1 | 0.2 | 73.6 | 0.1 | 1.6 |
| K-1 (Reference) | 24 | 1.1 | 0.7 | 10.8 | 17.2 | 2.3 | 63.3 | 3.6 | 1.0 |
| | 42 | 1.8 | 0.0 | 0.6 | 20.7 | 3.8 | 63.0 | 8.6 | 1.5 |

| | | | | | | | | | |
|---|---|---|---|---|---|---|---|---|---|
| In Table 1, Gly, DPI, Suc, Lac, Kes, LS, F-LS and UN denote glycerol, monosaccharides containing glucose and fructose, sucrose, lactose, 1-kestose, lactosucrose, fructosyl lactosucrose and other unknown saccharides, respectively. | | | | | | | | | |

As shown in Table 1, it was revealed that β-fructofuranosidase from *Bacillus* sp. V230, used in the present invention, formed 70% or more of lactosucrose, on a saccharide composition basis, for example, 71.0% (24 hours) and 73.6% (48 hours); and less than 1% of the total amount of by-products, 1-kestose and frucrosyl lactosucrose, for example, 0.1% (24 hours) and 0.3% (48 hours). While, it was revealed that β-fructofuranosidase from *Arthrobacter* sp. K-1, a reference, formed less than 65% of lactosucrose, on a saccharide composition basis, for example, 63.3% (24 hours) and 63.0% (48 hours); and remarkable amount of 1-kestose and frucrosyl lactosucrose, for example, the total of the by-products of 5.9% (24 hours) and 12.4% (48 hours).

### Example 2

### Production of a powdery lactosucrose high content saccharide

An aqueous solution containing 24% (w/w) of sucrose and 16% (w/w) of lactose was adjusted to pH 6.0, and then one unit/g-sucrose of the crude β-fructofuranosidase solution, prepared in Example 1-1, and 7.5% wet weight/dry-solid of the sucrose-unassimilable yeast cells, prepared in Example 1-2 were admixed with the aqueous solution and followed by the enzyme reaction at 30°C for 45 hours with controlling the pH 4.0 to 5.5 using 1 N of aqueous sodium hydroxide solution. After the reaction, saccharide composition was analyzed by HPLC, revealing that the reaction mixture contained 2.0% of glycerin, 2.1% of the total of glucose and fructose, 5. 4% of sucrose, 14.7% of lactose, 1.4% of 1-kestose, 73. 0% of lactosucrose, 0.1% of fructosyl lactosucrose, and 1.3% of other unknown saccharides. After heating the reaction mixture at 90°C for 30 minutes to inactivate the enzyme, the reaction mixture was filtrated using activated charcoal according to conventional method. After adjusting the resulting filtrate to 60°C, the pH was adjusted to 11 by adding 1.5%/dry-solid of calcium hydroxide and kept for 15 minutes, and then carbon dioxide was injected to the solution to bring the pH to 8 for saturating carbon dioxide. The resulting solution was filtrated according to conventional method, deionized using ion-exchange resin, concentrated, and spray-dried to make into a powdery lactosucrose high content saccharide containing 1.9% glycerin, 2.2% of the total of glucose and fructose, 5.5% of sucrose, 10.8% of lactose, 3.3% of lactulose, 1. 3% of 1-kestose, 73.5% of lactosucrose, 0.1% of fructosyl lactosucrose, and 1.4% of unknown saccharides, on a saccharide composition basis.

The product can be advantageously used in various fields such as foods and beverages including healthy foods and beverages, cosmetics, pharmaceuticals, feeds, and the like as a sweetener, hardly digestive sweetener, low-cariogenic sweetener, moisture-retaining agent, starch-retrogradation inhibiting agent and antiflatulent.

### Example 3

### Production of a syrupy lactosucrose high content saccharide

An aqueous solution containing 18% (w/w) of sucrose and 22% (w/w) of lactose was adjusted to pH 6.0, and then one unit/g-sucrose of the crude β-fructofuranosidase solution, prepared in Example 1-1, and 5.0% wet weight/dry-solid of the sucrose-unassimilable yeast cells, prepared in Example 1-2 were admixed with the aqueous solution and followed by the enzyme reaction at 32°C for 42 hours with controlling the pH 4.0 to 5.5 using 1 N of aqueous sodium hydroxide solution. After the reaction, saccharide composition was analyzed by HPLC, revealing that the reaction mixture contained 1.9% of glycerin, 0.1% of the total of glucose and fructose, 1.2% of sucrose, 22.6% of lactose, 0.3% of 1-kestose, 72.4% of lactosucrose, 0.1% of fructosyl lactosucrose, and 1.4% of other unknown saccharides. Yeast cells were removed from the resulting reaction mixture by filtration using a SF-membrane. Successively, high molecular components including β-fructofuranosidase were further removed from the filtrate by filtration using a UF-membrane. After filtrating the solution using activated charcoal, the resulting filtrate was concentrated *in vacuo* to obtain a concentrated solution with a concentration of about 80% (w/w). After adjusting the concentrate to about 25° C, about 3% (w/w) to the initial material lactose of powdery crystalline lactose was added to the concentrate for partially crystallizing lactose. After removing the crystallized lactose by conventional filtration, the resulting filtrate was deionized using ion-exchange resin and concentrated to make into a syrupy lactosucrose high content saccharide containing 2.2% glycerin, 0.1% of the total of glucose and fructose, 1.4% of sucrose, 10.1% of lactose, 0.4% of 1-kestose, 84.1% of lactosucrose, 0.1% of fructosyl lactosucrose, and 1.6% of unknown saccharides, on a saccharide composition basis.

The product can be advantageously used in various fields such as foods and beverages including healthy foods and beverages, cosmetics, pharmaceuticals, feeds, and the like as a sweetener, hardly digestive sweetener, low-cariogenic sweetener, moisture-retaining agent, starch-retrogradation inhibiting agent and antiflatulent.

### Example 4

### Production of a powdery high purity lactosucrose

An aqueous solution containing 20% (w/w) of sucrose and 20% (w/w) of lactose was adjusted to pH 6.0, and then one unit/g-sucrose of the crude β-fructofuranosidase solution, prepared in Example 1-1, and 5.0% wet weight/dry-solid of the sucrose-unassimilable yeast cells, prepared in Example 1-2 were admixed with the aqueous solution and followed by the enzyme reaction at 30°C for 30 hours with controlling the pH 4.0 to 5.5 using 1 N of aqueous sodium hydroxide solution. After the reaction, saccharide composition was analyzed by HPLC, revealing that the reaction mixture contained 2.0% of glycerin, 0.2% of the total of glucose and fructose, 1.6%of sucrose, 19. 2% of lactose, 0.6%of 1-kestose, 74.7% of lactosucrose, 0.1% of fructosyl lactosucrose, and 1.7% of other unknown saccharides. According to conventional method, yeast cells were removed from the resulting reaction mixture by filtration using a SF-membrane. Successively, high molecular components including β-fructofuranosidase were further removed from the filtrate by filtration using a UF-membrane. After filtrating the solution using activated charcoal, the resulting filtrate was concentrated *in vacuo* to obtain a concentrated solution with a concentration of about 60% (w/w). For increasing the purity of lactosucrose, the syrupy lactosucrose high content saccharide was subjected to a column chromatography using "AMBERLITE CR-1310" (Na-form), a strongly acidic cation-exchange resin, commercialized by Organo Corporation, Tokyo, Japan. The resin was packed into 10 jacketed stainless-steel columns with an internal diameter of 12.5 cm and the columns were connected in series to give the total bed length of 16 m. With keeping the column temperature to 40°C, 1.5% (v/v) to the total resin volume of the aforementioned syrup was injected to the column and fractionated by running water warmed to 40°C at SV 0.2, and then a lactosucrose high content fraction was collected with monitoring a saccharide composition of eluent by HPLC. According to conventional methods, the resulting fraction was filtrated, deionized using ion-exchange resin, decolored by activated charcoal, concentrated and spray-dried to make into a powdery high purity lactosucrose containing 0.1% of sucrose, 2.3% of lactose, 1.1% of 1-kestose, 96.0% of lactosucrose, 0.2% of fructosyl lactosucrose, and 0.3% of unknown saccharides, on a saccharide composition basis.

The product can be advantageously used in various fields such as foods and beverages including healthy foods and beverages, cosmetics, pharmaceuticals, feeds, and the like as a sweetener, hardly digestive sweetener, low-cariogenic sweetener, moisture-retaining agent, starch-retrogradation inhibiting agent and antiflatulent.

### Example 5

### Production of a granular high purity lactosucrose

About six parts by weight of aqueous pullulan solution, prepared by dissolving "PULLULAN PF-20", a pullulan product commercialized by Hayashibara Shoji Inc., Okayama, Japan, to give a final concentration of about 3% (w/v), was sprayed on 100 parts by weight of powdery high purity lactosucrose, obtained in Example 4, for the granulation. The resulting granules were sifted using a sieve with a sieve mesh of 1.7 mm to obtain granulated high purity lactosucrose. Comparison of particle size distribution of the granular high purity lactosucrose and material powdery high purity lactosucrose revealed that 85% or more of material powdery high purity lactosucrose was in particle diameter of 106 µm or lower, while 70% or more of granular high purity lactosucrose was in particle diameter of 150 µm or higher.

Since the product is in the form of granule, it shows a satisfactory fluidity and handleability. Further, it hardly forms flocks when dissolving in water. The product can be advantageously used in various fields such as foods and beverages including healthy foods and beverages, cosmetics, pharmaceuticals, feeds, and the like as a sweetener, hardly digestive sweetener, low-cariogenic sweetener, moisture-retaining agent, starch-retrogradation inhibiting agent and antiflatulent.

### Example 6

### Production of crystalline lactosucrose

According to conventional methods, lactosucrose high content fraction, obtained by the method in Example 4, was filtrated, deionized using ion-exchange resin, decolored using activated charcoal, and concentrated under a reduced pressure to give a moisture content of about 25% (w/w). The resulting concentrate was placed in a crystallizer, admixed with 1% (w/w) of seed crystal and gradually cooled to about 30°C in 24 hours. The resulting massecuite was centrifuged and crystalline lactosucrose pentahydrate was obtained in a yield of about 40%. The product is a crystalline lactosucrose with a purity of about 98% or higher and shows a satisfactory handleability and no substantial hygroscopicity even under the condition of high-temperature and humidity in summer. The product can be advantageously used in various fields such as foods and beverages, cosmetics, pharmaceuticals, and the like, particularly, uses for solid compositions comprising crystalline lactosucrose as a sweetener, hardly digestive sweetener and antiflatulent.

### Example 7

### Sweetener

One part by weight of crystalline lactosucrose and 0.05 part by weight of "αG-SWEET", α-glycosyl-stevioside commercialized by Toyo Sugar Refining Co., Ltd, Tokyo, Japan, were mixed to homogeneity and granulated using a granulator to make into a sweetener in a granule form. The product has a good sweetness and shows about 2-folds higher sweetness than sucrose, however, it has a reduced calorie to the sweetness of about a half of that of sucrose. The sweetener can be preferably used as a low-calorie sweetener for sweetening low-calorie foods and beverages, which are used for obese persons and diabetic patients restricted calorie intake. Further, since the sweetener hardly converted into acids and insoluble glucans by cariogenic bacteria, it can be preferably used for sweetening foods and beverages to inhibit dental caries. Furthermore, the sweetener can be preferably used as an agent for improving intestinal bacterial flora.

### Example 8

### Shaped sweetener

One part by weight of crystalline lactosucrose, obtained by the method in Example 6, and two parts by weight of powdered sucrose were mixed to homogeneity, and then appropriate amount of water was sprayed on the mixture. The resulting mixture was shaped into a shaped sweetener by conventional method. The product can be advantageously used for sweetening coffee, black tea, and the like as a shaped sweetener with a function of regulating intestine, which can be used for improving intestinal bacterial flora.

### Example 9

### Chocolate

Forty parts by weight of cacao paste, 10 parts by weight of cacao butter, 20 parts by weight of sucrose, and 30 parts by weight of crystalline lactosucrose, obtained by the method in Example 6, were mixed and the resulting mixture was refined using a refiner. Successively, the mixture was placed into a conche and kneaded at 50° C for 2 days. During the kneading, 0.5 parts by weight of lecithin was admixed with the mixture and mixed sufficiently for dispersion. Successively, the mixture was cooled to 30°C using a temperature controller, poured into a mold just before solidifying butter. Then, air bubbles were removed from the mixture using a vibrator and the mixture was solidified by leaving in a cooling tunnel controlled at 10° C. The resulting chocolate was unmolded and packed to make into a product. The product shows no hygroscopicity and satisfactory color, gloss, and internal texture. Also, the product melts easily in a mouth and gives fine sweetness and flavors. The product is a chocolate which can be preferably used for regulating the function of intestine.

### Example 10

### Chewing gum

Three parts by weight of gum base was softened by heating and melting, and then admixed with four parts by weight of anhydrous crystalline maltitiol, commercialized by Hayashibara Shoji Inc., Okayama, Japan, three parts by weight of crystalline lactosucrose, obtained by the method in Example 6, one part by weight of hydrous crystalline trehalose , and appropriate amounts of flavor and colorings. The mixture was kneaded by a roll, shaped and packed to make into chewing gum. The product has a satisfactory texture and flavor. Further, the product is preferable as a chewing gum for regulating the functions of intestine.

### Example 11

### Beverage

One hundred parts by weight of yoghurt, 50 parts by weight of a powdery high purity lactosucrose, obtained by the method in Example 4, 10 parts by weight of trehalose, 0.25 part by weight of yoghurt flavor and 0.1 part by weight of lemon essence were dissolved into water to give the total weight of 1,000 parts by weight, and then made into a yoghurt beverage by conventional method. The product is rich in flavor and has a function of regulating intestine for improving intestinal bacterial flora.

### Example 12

### Agent for tube feeding

Ten parts by weight of crystalline lactosucrose, obtained by the method in Example 6, 10 parts by weight of trehalose, 1.1 parts by weight of glycine, one part by weight of sodium glutamate, 0.4 part by weight of calcium lactate, 0.1 part by weight of magnesium carbonate, 0.01 part by weight of thiamine and 0.01 part by weight of riboflavin were mixed to make into a composition. Twenty-four grams each of the composition was packed into a laminate aluminum pouch, and the pouch was heat-sealed to make into a product. A nutraceutical solution can be prepared by dissolving a pouch of the product into about 33 to 500 milliliters of water and the resulting solution can be administrated by tube feeding to nasal cavity, stomach, intestine, and the like. The product can be advantageously used as a non-oral neutraceutical solution for domestic animals as well as human. Further, the product has a function of regulating intestine for improving intestinal bacterial flora.

### Example 13

### Sugarcoated tablet

One hundred and fifty mg of a raw tablet, used as core, was sugar-coated to give about 230 mg of sugar-coated tablet by using solution 1 consisting of 45 parts by weight of crystalline lactosucrose, obtained by the method in Example 6, two parts by weight of pulullan (average molecular weight of 200,000), 30 parts by weight of water, 25 parts by weight of talc, and three parts by weight of titanic oxide. Subsequently, the resulting tablet was further sugar-coated by using solution 2 consisting of 65 parts by weight of crystalline lactosucrose, one part by weight of pullulan, and 34 parts by weight of water. Furthermore, the resulting sugar-coated tablet was polished using wax to produce a sugar coated tablet having a satisfactory gloss and appearance. The sugarcoating can be carried out efficiently to produce the product. The product has a superior shock tolerance and keeps the high quality for a long period of time. Further, the product has a function of improving bacterial flora in intestine.

### INDUSTRIAL APPLICABILITY

As described above, according to the process for producing a lactosucrose high content saccharide of the present invention, a lactosucrose high content saccharide comprising 70% or more of lactosucrose, on a saccharide composition basis, can be produced from sucrose and lactose by a reaction using β-fructofuranosidase and a sucrose-unassimilable yeast in combination without using purification procedures such as column chromatography. Further, since the lactosucrose high content saccharide obtainable by the process of the present invention comprises by-products, 1-kestose and fructosyl lactosucrose, in a low content, less than 3% on a saccharide composition basis, of the total of them, a high purity lactosucrose can be easily prepared in a high yield by purifying it as material using chromatography.

Since the lactosucrose high content saccharide or the high purity lactosucrose, obtainable by the present invention, contains 70% or more or 90% or more, on a saccharide composition basis, of lactosucrose which is an effective component for exhibiting functions, they are able to exhibit satisfactory functions inherent to lactosucrose such as low-digestivity, bifidobacteria growth-promoting activity, low-cariogenicity, and moisture-retaining ability, in a relatively low amount in comparison with a conventional saccharide comprising lactosucrose with a low content of lactosucrose. Therefore, the lactosucrose high content saccharide and the high purity lactosucrose, obtainable by the present invention, enable the selection from wide range formulae for incorporating them with other materials, in the case of producing various compositions such as foods and beverages, cosmetics, pharmaceuticals, feeds, and the like by incorporating them.

In addition, crystalline lactosucrose pentahydrate, produced by crystallizing a supersaturated solution of lactosucrose including the lactosucrose high content saccharide and the high purity lactosucrose, has a substantial non-hygroscopicity, satisfactory fluidity and handleability with out fear of adhesion and solidification. Therefore, crystalline lactosucrose pentahydrate obtainable by the present invention can be advantageously used for various solid compositions. The present invention, having these outstanding functions and effects, is a significantly important invention that greatly contributes to this art.

## Claims

1. A lactosucrose high content saccharide, comprising 70% or more of lactosucrose and less than 3% of the total amount of 1-kestose and fructosyl lactosucrose, on a saccharide composition basis.

2. The lactosucrose high content saccharide of claim 1, further containing one or more other saccharides selected from the group consisting of glycerin, glucose, fructose, sucrose, lactose and lactulose.

3. A process for producing the lactosucrose high content saccharide of claim 1 or 2, comprising the steps of:
(a) allowing β-fructofuranosidase, derived from a microorganism belonging to the genus *Bacillus*, and sucrose - unassimilable yeast to contact with an aqueous solution containing sucrose and lactose to obtain a reaction mixture comprising 70% or more of lactosucrose and less than 3% of the total amount of 1-kestose and fructosyl lactosucrose;
(b) optionally, isomerizing the concomitant lactose into a mixture of lactose and lactulose; and
(c) collecting the resulting lactosucrose high content saccharide.

4. The process of claim 3, wherein said β-fructofuranosidase derived from a microorganism of the genus *Bacillus* is a recombinant enzyme.

5. The process of claim 3 or 4, wherein said microorganism of the genus *Bacillus* is *Bacillus* sp. V230 (FERM BP-5054).

6. The process of any one of claims 3 to 5, wherein said sucrose-unassimilable yeast is *Saccharomyces cerevisiae* ATCC56741 or ATCC56742.

7. The process of any one of claims 3 to 6, wherein the ratio of sucrose and lactose, which are materials for lactosucrose-forming reaction, is 1:0.65 to 1.3.

8. The process of any one of claims 3 to 7, wherein the pH of the reaction mixture for forming lactosucrose is controlled in a range of 4.0 to 5.5.

9. A process for producing a high purity lactosucrose, comprising the steps of:
(a) subjecting the lactosucrose high content saccharide of claim 1 or 2 to a chromatography using a resin; and
(b) collecting fractions containing 90% or more of lactosucrose, on a saccharide composition basis.

10. A process for producing a high purity lactosucrose powder, comprising the steps of:
(a) subjecting the lactosucrose high content saccharide of claim 1 or 2 to a chromatography using a resin;
(b) collecting fractions containing 90% or more of lactosucrose, on a saccharide composition basis;
(c) concentrating the resulting saccharide solution; and
(d) spray-drying the resulting concentrate.

11. The process of claim 9 or 10, further containing a step of granulating the resulting powder.

12. A process for producing crystalline lactosucrose, comprising the steps of:
(a) preparing a supersaturated lactosucorse solution using the lactosucrose high content saccharide of claim 1 or 2, or the high purity lactosucrose obtainable by the process of claim 9;
(b) crystallizing lactosucrose from the supersaturated lactosucrose solution; and
(c) collecting the resulting crystalline lactosucrose.

13. The process of claim 12, wherein said crystalline lactosucrose is in the form of crystalline lactosucrose pentahydrate.

14. A solid composition prepared by incorporating crystalline lactosucrose obtainable by the process of claim 12 or 13.

15. The solid composition of claim 14, wherein other crystalline saccharide(s) is incorporated in an amount of 0.01 to 100-folds by weight to the crystalline lactosucrose.

16. The solid composition of claim 14 or 15, which is in the form of a food and beverage, cosmetic or pharmaceutical.
